# EUROPEAN PATENT APPLICATION

(11) **EP 4 212 617 A1**
(43) Date of publication of application: **19.07.2023**
(21) Application number: 21799165.2
(22) Date of filing: 25.06.2021
(51) Int. Cl.: C12N 5/078, A01N 1/02, A61K 35/19, A61P 29/00, A61P 9/10

(54) **METHOD FOR OBTAINING PLATELET-DERIVED MITOCHONDRIA AND USE THEREOF**

(30) Priority: 10.09.2020 KR 20200116297
(71) Applicant: Paean Biotechnology Inc., Daejeon 34013 (KR)
(72) Inventor: KANG, Young-cheol, Wonju-si, Gangwon-do 26463 (KR); HAN, Kyuboem, Daejeon 34119 (KR); HA, Jong-Cheon, Daejeon 34048 (KR)
(74) Representative: HGF
(86) International application number: PCT/KR2021/008030
(87) International publication number: WO 2022/055090

(57) **Abstract**

Provided are a method for obtaining active mitochondria by freezing platelets and a use of isolated mitochondria, and more specifically, to a method for obtaining mitochondria by thawing platelets after freezing the same and a pharmaceutical composition containing the mitochondria obtained by the method as an active ingredient. The present disclosure, by providing a method for obtaining mitochondria by thawing platelets in a frozen state in a preservation solution, not only enables to obtain mitochondria in which the activity is stably maintained, but also enables to provide a commercial value for donor platelets that are discarded after refrigeration for a short period of time. Additionally, the pharmaceutical composition containing platelet-derived mitochondria obtained by the above method may be effectively used to treat various diseases caused by mitochondrial dysfunction.

## Description

### BACKGROUND

The present disclosure relates to a method for obtaining active mitochondria from a frozen platelet and a use of the obtained mitochondria. More particularly, it is directed to a method for obtaining active mitochondria from a frozen platelet and a pharmaceutical composition containing the mitochondria therefrom as an active ingredient and a use thereof.

Mitochondria are eukaryotic organelles involved in the synthesis and regulation of adenosine triphosphate (ATP), which is an intracellular energy source. Mitochondria are associated with the control of various metabolic pathways *in vivo*, such as cell signaling, cell differentiation, and apoptosis, as well as cell cycle and cell growth.

It has been reported that the decrease in energy production due to the deterioration of mitochondrial functions causes various diseases. When the function of the electron transport chain reaction is decreased due to a mutation in the genome and proteome of mitochondria, it results in a decrease of ATP production, excessive production of reactive oxygen species, deterioration of calcium regulation function, *etc.* In this case, mitochondria may undergo a change in its membrane permeability and induce an abnormal function of apoptosis, which may cause cancer and intractable diseases accordingly.

It has been reported that examples of human diseases caused by mitochondrial dysfunction include mitochondria-related genetic diseases, autoimmune inflammatory diseases (e.g., rheumatoid arthritis), ischemic diseases, infectious diseases, heart diseases, muscular diseases, degenerative diseases (e.g., Parkinson's disease and Alzheimer's disease), and occurrence and metastasis of various cancers, etc.

In order to treat such mitochondrial dysfunction-related diseases, studies on substances that can be used to protect mitochondria or restore mitochondrial dysfunction or efficient target delivery of these substances have been underway. However, the range of treatment has been somewhat limited or there have been a problem of side effects, and a breakthrough treatment for mitochondria-related diseases has not yet been developed.

The cell membrane permeability and cell-cell mobility of mitochondria have been confirmed as mitochondrial origin and a means of signaling. Using such mobility, studies on mitochondrial transplantation to replace damaged mitochondria with normal mitochondria are underway in various disease models, and its safety and efficacy have been confirmed to the clinical stage in heart diseases (McCully et al., Clin Trans Med., 5(16): 1-13, 2016).

Isolation of mitochondria for such mitochondrial transplantation is being conducted from autologous tissues and muscles through additional surgery or massively cultured human cells. However, such an isolation method using human resources faces a problem that it is difficult to commercialize the isolated mitochondria as a therapeutic agent due to the burden of surgery and limited resources by the limitation of cell culture.

In addition, in the blood, which is a human resource that is easy to donate and transplant, mitochondria exist in all of blood cell components except red blood cells, attempts to treat diseases using the mitochondria isolated from platelets have not been reported thus far.

Platelets are the smallest blood cell component in the blood and the number of platelets is in the range of 150,000 to 400,000 per 1 µL of blood in a normal person. Platelets move around blood vessels so as to maintain the blood flow without loss of blood and react when there occurs a bleeding, and they play a role in aggregation and hemostasis. It is also known to contain numerous proteins, cytokines, and biologically active factors, thus being able to initiate and regulate wound healing.

Platelets are produced in the bone marrow and survive for about 7 days *in vivo.* However, once the blood is collected, the storage period of platelets is generally shorter than those of other blood products, and it is currently known that platelets can only be stored in a stirrer at room temperature for up to 5 days. One of the reasons for the further deterioration of the storage stability of platelets compared to other blood products such as red blood cells is because the metabolism by the mitochondria present in these platelets occurs continuously, and as the retention time elapses, ATP decreases along with a decrease in the pH. In particular, when platelets are stored at a low temperature so as to inhibit metabolism, they fail to maintain their cytoskeleton and are deformed to induce aggregation, and the functions of platelets cannot be expected when thawed after freezing.

In addition, it takes about 24 to 48 hours to isolate platelets from the blood, and the actual shelf-life of platelets is only 3 to 4 days, excluding the isolation time. These platelet preparations are used for the prevention or treatment of bleeding in patients with a sudden decrease in platelets, and thus, their uses are limited. Therefore, 25% of the platelets collected from the blood are discarded after the expiration date.

Therefore, the effect by the development of a technology capable of stably isolating active mitochondria for use in disease treatment from platelets, which are easier to obtain than autologous tissues and stem cells but have limited storage and use, is expected to be very large.

### DISCLOSURE

### TECHNICAL PROBLEM

Under the circumstances, the present inventors have made an extensive effort to develop a breakthrough method for obtaining mitochondria for a stable supply of mitochondria for transplantation treatment as part of the treatment of mitochondria-related diseases. As a result, it was confirmed that mitochondria, which were obtained from platelets which were secured and stored frozen by thawing, not only stably maintain the membrane potential, but also have an activity that can excellently treat mitochondrial dysfunction-related diseases, specifically inflammatory diseases, thereby completing the present disclosure.

### TECHNICAL SOLUTION

For the achievement of those purposes described above, in accordance with an exemplary embodiment, the present disclosure provides a method for obtaining platelet-derived mitochondria, which includes freezing or storing platelets isolated from blood; thawing the frozen platelets; and disrupting the thawed platelets.

In accordance with another exemplary embodiment, the present disclosure also provides a pharmaceutical composition for preventing or treating mitochondrial dysfunction-related diseases which contains the platelet-derived mitochondria obtained by the above method as an active ingredient.

### ADVANTAGEOUS EFFECTS

The method for obtaining mitochondria from frozen platelets according to the present disclosure not only may facilitate platelet disruption thus enabling to obtain mitochondria with stable activity, but also may provide a commercial value for donor platelets that are discarded after refrigeration for a short period of time. In addition, the pharmaceutical composition containing platelet-derived mitochondria obtained by the above method is expected to have high commercial applicability because it may be widely applied to the treatment of various diseases caused by mitochondrial dysfunction.

### BRIEF DESCRIPTION OF THE DRAWINGS

Exemplary embodiments can be understood in more detail from the following description taken in conjunction with the accompanying drawings, in which:
FIG. 1a shows a flow chart, which illustrates a method for obtaining platelet-derived mitochondria according to the present disclosure;
FIG. 1b shows an image of fluorescent-stained platelet-derived mitochondria which were obtained according to the present disclosure (in particular, the marker used (Mitotracker Green) is a fluorescent dye that accumulates in mitochondria);
FIG. 2 shows graphs which illustrate the comparison results between the method for obtaining platelet-derived mitochondria according to the present disclosure, where freezing and thawing were additionally performed, and the existing method for mitochondria isolation using a vortex and a syringe (in which F represents freezing and T represents thawing);
FIG. 3 shows a graph which illustrates the yield of mitochondria isolation according to a platelet isolation solution and freezing conditions (in which LN₂ represents liquid nitrogen; additionally, SHE represents a mixed sucrose-HEPES-EGTA preservation solution, and TTG represents a mixed trehalose-Tris-glycine preservation solution);
FIG. 4 shows graphs which illustrate the yield of mitochondria isolation and the amount of ATP retained according to the freezing storage period of platelets;
FIG. 5 shows a graph which illustrates the inhibitory effect of the platelet-derived mitochondria, which were obtained according to the present disclosure, against the generation of reactive oxygen species induced by lipopolysaccharide (LPS) in THP-1 human monocytes;
FIG. 6 shows a graph which confirms the survival rate in an LPS-induced inflammation animal model administered with platelet-derived mitochondria obtained according to the present disclosure; and
FIG. 7 shows a graph which illustrate the yield of mitochondria isolated from platelet according to the thawing conditions.

### DETAILED DESCRIPTION OF EMBODIMENTS

Hereinafter, specific embodiments will be described in detail with reference to the accompanying drawings.

### Method for storing mitochondria

In an aspect, the present disclosure provides a method for stably storing mitochondria by freezing platelets.

### Method for obtaining platelet-derived mitochondria

In an aspect, the present disclosure provides a method for obtaining platelet-derived mitochondria, which includes thawing frozen platelets and disrupting the thawed platelets.

As used herein, the term "platelet" refers to a blood corpuscle component that plays an important role in the hemostatic mechanism of a living body, and it has a diameter of 2 µm to 3 µm, and the number of platelets per 1 µL of blood in a normal person is 150,000 to 400,000. In addition, it is known that platelets are viscous, change their shape depending on conditions, and cause a series of a coagulation reaction by releasing intracellular components while simultaneously adhering and aggregating to damaged tissues.

As used herein, the term "mitochondria" refers to an organelle of a eukaryotic cell involved in the synthesis and regulation of adenosine triphosphate (ATP), which is an intracellular energy source, and it is associated with the control of various metabolic pathways *in vivo* (e.g., cell signaling, cell differentiation, apoptosis, cell cycle, and cell growth).

The mitochondria may be obtained from a mammal or may be obtained from a human. Specifically, the mitochondria may be obtained from platelets among blood corpuscle components. In addition, the mitochondria may be obtained from autologous or heterologous platelets.

As used herein, the term "blood corpuscle" refers to a cellular component such as red blood cells, white blood cells, and platelets contained in blood; plasma and blood corpuscles may be isolated from the blood by centrifugation; and the blood corpuscle components (e.g., red blood cells, white blood cells, and platelets) sink to the bottom of the centrifuge tube.

Any blood corpuscle may be used as long as it contains mitochondria without any particular limitation thereto.

In addition, as the blood containing platelets for mitochondria isolation, as long as it is not particularly limited, allogenic or xenogenic blood compared to that of a transplanter may be used, and autologous blood may be used.

The platelet-derived mitochondria may be obtained by using various known methods, for example, a method using a specific buffer solution or using a potential difference, magnetic field, etc.

Specifically, the mitochondria may be obtained by thawing frozen platelets, disrupting, and centrifugation. In addition, the method for obtaining mitochondria may be proceeded to include: 1) freezing platelets; 2) thawing platelets; and 3) disrupting the thawed platelets.

In a specific embodiment, the method for obtaining mitochondria may be proceeded to include: subjecting a whole blood sample to a first centrifugation so as to obtain platelet-rich plasma (PRP) containing platelets; subjecting the PRP to a second centrifugation so as to obtain a platelet pellet; resuspending the platelet pellet in a buffer solution and freezing; thawing the frozen platelets; disrupting the thawed platelets; and removing the lysate excluding mitochondria. In particular, the centrifugation time and the centrifugation speed may be increased as the centrifugation proceeds from the first centrifugation to the second centrifugation.

More specifically, the step of removing the lysate excluding mitochondria may be proceeded to include: subjecting the lysed platelets to a third centrifugation so as to obtain a supernatant; and subjecting the obtained supernatant to a fourth centrifugation so as to obtain and purify mitochondria. In particular, the centrifugation time and the centrifugation speed may be increased as the centrifugation proceeds from the third centrifugation to the fourth centrifugation.

The isolation of mitochondria by the above obtaining method may be performed through various centrifugal forces (g force) and changes in time used in differential centrifugation known in the art. Specifically, the first centrifugation may be performed at a speed of 100 ×g to 1,000 ×g, 200 ×g to 700 ×g, or 300 ×g to 600 ×g. In addition, the second centrifugation may be performed at a speed of 100 ×g to 3,000 ×g, 500 ×g to 2,800 ×g, or 800 ×g to 2,500 ×g. In addition, the third centrifugation may be performed at a speed of 800 ×g to 4,000 ×g, 1,000 ×g to 3,000 ×g, or 1,200 ×g to 2,600 ×g. In addition, the fourth centrifugation may be performed at a speed of 100 ×g to 20,000 ×g, 500 ×g to 18,000 ×g, or 800 ×g to 15,000 ×g.

In addition, the time during which the centrifugation is performed may be performed for 1 to 50 minutes, and may be appropriately adjusted according to the number of centrifugations, the content of the sample, *etc.*

In addition, the third centrifugation may be performed at room temperature of 1°C to 30°C, or refrigeration temperature of 2°C to 8°C, preferably at a temperature of 3°C to 5°C, and by obtaining the supernatant through the third centrifugation, it is possible to remove residual cells, cell membrane lysate, etc. (i.e., debris) from the lysed platelets.

In addition, the fourth centrifugation may be performed at a room temperature of 1°C to 30°C, or refrigeration temperature of 2°C to 8°C, and preferably at a temperature of 3°C to 5°C, and it is possible to obtain mitochondria from the platelet lysate, in which debris has been removed, by obtaining a pellet through the fourth centrifugation and resuspending the pellet.

In addition, mitochondria may be obtained and purified by centrifugation, or using a filter, a mesh, or beads when removing the platelet lysate. For example, mitochondria may be obtained and purified using a nylon mesh filter having a pore size of 0.1 µm to 40 µm, beads bound by an immunological method (e.g., antibody-conjugated magnetic beads), etc.

Meanwhile, the method may further include washing the obtained mitochondria by a fifth centrifugation. Specifically, the fifth centrifugation may be performed at a speed of 100 ×g to 20,000 ×g, 500 ×g to 18,000 ×g, or 800 ×g to 15,000 ×g. In addition, the fifth centrifugation may be performed at room temperature of 1°C to 30°C, or refrigeration temperature of 2°C to 8°C, and preferably at a temperature of 3°C to 5°C. In addition, the centrifugation may be performed for 1 to 50 minutes, but is not limited thereto.

In addition, a pharmaceutically acceptable sugar may be used for stabilizing the obtained platelet-derived mitochondria. Specifically, the pharmaceutically acceptable sugar may be a saccharide such as sucrose, mannitol, or trehalose, but is not limited thereto. In addition, although not limited thereto, a pharmaceutically acceptable Tris, HEPES, phosphate, etc. may be used as a pH buffer.

Meanwhile, after obtaining the platelet-derived mitochondria, additives (e.g., chelators, antioxidants, etc.) may be used to remove damage caused by ion leakage and inhibit oxidative stress, and it may be used by including, without limitation, reagents well known in the art. For example, the additives may be EDTA, EGTA, citrate, glycine, taurine, ATP, *etc.*, but are not limited thereto.

In the method of obtaining mitochondria of the present disclosure, the frozen platelets may be the platelets which were obtained from a subject and then frozen.

The platelets may be obtained from the same subject or from another subject and frozen. The subject may be a mammal, preferably a human.

In addition, the platelets may be frozen platelets isolated from blood, cultured blood, bone marrow, or cultured megakaryocytes. Any platelet which contains mitochondria may be used and there is no particular limitation thereto.

The freezing is not limited thereto, but may be performed at a temperature of -1°C or below. Specifically, the freezing may be performed at a temperature range of -5°C to -200°C, -15°C to -180°C, -25°C to -160°C, -40°C to - 140°C, -55°C to -120°C, -60°C to -100°C, or - 70°C to -90°C.

The freezing may be performed using liquid nitrogen (LN₂) or a refrigerating device. Specifically, the freezing may be performed by quick freezing using LN₂ in the refrigeration or freezing, or using a refrigeration device (e.g., a deep freezer, a freezer, a freezing container, etc.). The freezing is not particularly limited as long as it is a method capable of refrigeration or freezing platelets.

The period of cryopreservation of the frozen platelets includes all from the period of immediately after quick freezing or freezing to the period of limitation for maintaining the frozen state. That is, as long as mitochondria having normal activity may be isolated from frozen platelets, the period of cryopreservation is not particularly limited.

In addition, the platelet may be frozen rapidly, moderately, or slowly. Then, the platelet may be slowly frozen using a freezing container. Then, the platelet may be frozen by lowering -5°C to -0.1°C per minute. Specifically, the platelet may be frozen by lowering about -5°C, about -4°C, about -3°C, about -2°C, about -1°C, or about -0.5°C per minute. Preferably, the platelet may be frozen by lowering about - 1°C per minute.

According to an example of the present invention, it was confirmed that the slow-frozen platelets had higher mitochondrial yield and activity upon thawing compared to the quick-frozen platelets.

In addition, although not limited thereto during the period of freezing or cryopreservation, platelets may be stored after quick freezing or freezing in a mixed preservation solution (*e.g.*, a sucrose-HEPES-EGTA (SHE) solution, a trehalose-Tris-glycine (TTG) solution, a trehalose-Tris-glycine-EDTA (TTGE) solution, *etc*.). The preservation solution is not particularly limited as long as mitochondria having normal activity may be obtained from platelets which were stored frozen for a predetermined period of time.

In addition, any additives effective for increasing the isolation yield of mitochondria obtained from platelets and capable of maintaining functions (e.g., antioxidants, ATP, *etc.*) may be further added to the preservation solution.

In the method for obtaining mitochondria of the present disclosure, disruption of the frozen platelets may be performed after thawing.

The thawing may be performed at room temperature. Specifically, the thawing may be performed at 0°C to 37°C, 4°C to 30°C, 8°C to 28°C, 10°C to 27°C, 12°C to 26°C, or 15°C to 25°C, but the thawing is not particularly limited as long as the frozen platelets are melted and become non-cryofrozen. Specifically, the thawing may be performed at about 0°C, about 1°C, about 2°C, about 3°C, about 4°C, about 5°C, about 6°C, about 7°C, about 8°C, about 9°C, about 10°C, about 11°C, about 12°C, about 13°C, about 14°C, about 15°C, about 16°C, about 17°C, about 18°C, about 19°C, about 20°C, about 21°C, about 22°C, about 23°C, about 24°C, about 25°C, about 26°C, about 27°C, about 28°C, about 29°C, about 30°C, about 31°C, about 32°C, about 33°C, about 34°C, about 35°C, about 36°C, or about 37°C.

In addition, the thawing may be performed for 1 minute to 1 hour, 1 minute to 50 minutes, 1 minute to 40 minutes, 1 minute to 30 minutes, 1 minute to 20 minutes, 1 minute to 10 minutes, or 1 minute to 5 minutes, but the thawing time is not limited thereto.

In the method for obtaining mitochondria of the present disclosure, the frozen and thawed platelets may be further disrupted by a physical or chemical method. Specifically, the disruption may be performed by a physical method using a shear force applied through a vortex and/or syringe. As the syringe, although not limited thereto, a syringe of 27G or 29G may be used. In addition, the method may be performed by devices using ultrasonic waves, pressurization method, etc. (e.g., a sonicator, a homogenizer, a high-pressure homogenizer, etc.). In addition, the disruption may be performed by a chemical method using a protease, a detergent, etc. The disruption is not particularly limited as long as mitochondria may be isolated by disrupting platelets.

In the method for obtaining mitochondria, freezing and thawing of the platelets may be further performed at least once before the disruption of the platelets. The number of times of freezing and thawing is not particularly limited as long as mitochondria isolated from the platelets have normal activity.

### Use of platelet-derived mitochondria

In another aspect, the present disclosure provides a pharmaceutical composition for preventing or treating diseases, which contains the platelet-derived mitochondria obtained by the above obtaining method as an active ingredient. In particular, the use of the pharmaceutical composition may be to prevent or treat mitochondria-related diseases.

As used herein, the term "mitochondria-related diseases" collectively refers to diseases involving deterioration of mitochondrial functions or mitochondrial dysfunction due to genetic, environmental, or unknown causes. For example, these diseases include genetic diseases (e.g., Leber's hereditary optic neuropathy (LHON), Leigh syndrome, myoclonic epilepsy associated with ragged-red Fibers (MERRF), and mitochondrial myopathy, encephalopathy, hyperlactacidemia, mitochondrial myopathy, encephalopathy, lactacidosis, stroke (MELAS), etc.); degenerative neurological diseases (e.g., Parkinson's disease and Alzheimer's disease); metabolic diseases (e.g., diabetes and obesity); inflammatory diseases (e.g., sepsis, rheumatoid arthritis, etc.).

The pharmaceutical composition of the present disclosure containing platelet-derived mitochondria as an active ingredient, although not limited thereto, may prevent or treat any one disease selected from the group consisting of sepsis (KR 10-2019-0050017 A), cancer (KR 10-2126199 B1), heart disease, rheumatoid arthritis (KR 10-2019-0094124 A), ischemic disease (KR 10-2019277 B1), infectious disease (KR 10-2018-0054523 A), Parkinson's disease, Alzheimer's disease, and muscle disease (KR 10-2019-0090754 A).

Specifically, the cancer, although not limited thereto, may be stomach cancer, liver cancer, lung cancer, colon cancer, breast cancer, prostate cancer, ovarian cancer, pancreatic cancer, uterine cervical cancer, thyroid cancer, laryngeal cancer, acute myeloid leukemia, brain tumor, neuroblastoma, retinoblastoma, head and neck cancer, salivary gland cancer, lymphoma, etc.

Additionally, the ischemic disease, although not limited thereto, may be severe limb ischemia, ischemic stroke, ischemic heart disease, ischemic colitis, *etc.* In particular, the ischemic disease may be a disease caused by mitochondrial abnormality, and it includes all of the ischemic cell disorders caused by deterioration in mitochondrial functions.

Additionally, the infectious disease, although not limited thereto, may be hepatitis B, hepatitis C, human papilloma virus (HPV) infection, cytomegalovirus infection, viral respiratory disease, influenza virus infection, etc.

Additionally, the muscle disease, although not limited thereto, may be MELAS syndrome, MERRF syndrome, Keams-Sayre syndrome, myopathy, encephalomyopathy, myasthenia, myasthenia gravis, amyotrophic lateral sclerosis, muscular dystrophy, muscular atrophy, hypotonia, muscle weakness, muscle stiffness, *etc.* In particular, the muscle disease may include any muscle cell disorders caused by decreased mitochondrial function.

Furthermore, the use of the pharmaceutical composition may be specifically for the prevention or treatment of chronic inflammatory disease, acute inflammatory disease, ischemic disease, neurological disease, heart disease, muscular disease, degenerative disease, metabolic disease, fibrotic disease, joint disease, eye disease, hair loss, and immune-related disease.

As used herein, the term "prevention" refers to any action that inhibits the occurrence of mitochondria-related diseases or delays the onset of mitochondria-related diseases through the administration of the pharmaceutical composition. As used herein, the term "treatment" refers to any action that improves or beneficially changes the symptoms of mitochondria-related diseases through the administration of the pharmaceutical composition.

The pharmaceutical composition may comprise a therapeutically effective amount or a pharmaceutically effective amount.

As used herein, the term "therapeutically effective amount" or "pharmaceutically effective amount" refers to an amount of a composition effective for preventing or treating a target disease, and also refers to an amount which is sufficient to treat a disease with a reasonable benefit/risk ratio applicable to a medical treatment, and does not cause side effects. The level of the effective amount may be determined according to factors including health conditions of a patient, kinds of disease, severity, a drug activity, sensitivity to drugs, an administration method, an administration time, an administration route, an excretion rate, a treatment duration, a combination, or concurrently used drugs, and other factors well known in the medical field.

In the pharmaceutical composition of the present disclosure containing platelet-derived mitochondria as an active ingredient, the mitochondria, although not limited thereto, may be contained at a concentration of 0.1 µg/mL to 500 µg/mL, 0.2 µg/mL to 450 µg/mL, or 0.5 µg/mL to 400 µg/mL. By containing the mitochondria in the above range, it becomes easy to adjust the mitochondrial dose when the pharmaceutical composition is administered, the degree of symptom improvement in patients' mitochondria-related diseases, specifically inflammatory diseases may be further improved. In particular, the dose of mitochondria may be quantified by quantifying the membrane protein of the platelet-derived mitochondria. Specifically, the isolated mitochondria may be quantified by the Bradford protein assay (James D. McCully, J. Vis. Exp. 2014; (91): 51682).

In particular, as the pharmaceutical composition according to the present disclosure, mitochondria may be administered in an amount of 0.01 mg/kg to 5 mg/kg, 0.1 mg/kg to 4 mg/kg, or 0.25 mg/kg to 2.5 mg/kg based on the body weight of the subject for one administration, but is not limited thereto. That is, it is most preferred from the aspect of cell activity that, as the pharmaceutical composition, mitochondria be administered in an amount within the above range based on the body weight of the subject in which the mitochondria-related disease is caused.

Additionally, the pharmaceutical composition may be administered 1 to 10 times, 3 to 8 times, or 5 to 6 times, and preferably 5 times. In particular, the administration interval may be 1 to 7 days or 2 to 5 days, and preferably at intervals of 3 days.

As used herein, the term "administration" refers to the process of a certain material into a subject by any suitable method, and the administration route of the composition may be administered via any general route as long as the composition may reach the target tissue. Preferably, it may parenteral administration, but although not limited thereto, e.g., intraperitoneal administration, intravascular administration, muscular administration, mucosal administration, subcutaneous administration, intradermal administration, ocular administration, etc.

Additionally, the pharmaceutical composition according to the present disclosure may be administered to humans or other mammals, in which mitochondria-related diseases may be caused or which are suffering from such disease or disorder. Additionally, the pharmaceutical composition may be an injection that may be administered intravenously or may be an injection that may be administered locally, and preferably it may be an injection preparation.

Accordingly, the pharmaceutical composition according to the present disclosure may be prepared as an injection that is physically and chemically very stable by adjusting the pH using a buffer that may be used for injection so as to secure product stability according to the distribution of injection prescriptions.

Specifically, the pharmaceutical composition of the present disclosure may contain water for injection. The water for injection is distilled water which is prepared to dissolve a solid injection or to dilute a water-soluble injection, and it may be a glucose injection, xylitol injection, D-mannitol injection, fructose injection, physiological saline, dextran 40 injection, dextran 70 injection, amino acid injection, Ringer's solution, lactic acid-Ringer's solution, Tris with a pH ranging from 3.5 to 8, HEPES, citric acid, phosphate buffer, or sodium dihydrogen phosphate-citrate buffer, *etc.*

Additionally, the pharmaceutical composition of the present disclosure may contain a stabilizing agent or solubilizing agent. For example, the stabilizing agent may be pyrosulfite or ethylene diaminetetraacetic acid, and the solubilizing agent may be hydrochloric acid, acetic acid, potassium phosphate hydroxide, potassium hydrogen carbonate, potassium carbonate, or Tris.

In a specific embodiment, the pharmaceutical composition may contain a mixed preservative solution (e.g., a trehalose-Tris-glycine (TTG) solution) and may be commonly used in pharmaceutically acceptable pharmaceutical preparations. As an additive of the pharmaceutical composition, antioxidants, ATP, magnesium, etc., which are effective for maintaining and active mitochondrial function, may be contained.

In a further aspect, the present disclosure provides a use of the platelet-derived mitochondria obtained from the method, for the prevention or treatment of the mitochondrial dysfunction-related diseases.

Then, the mitochondrial dysfunction-related disease, prevention, and treatment are aforementioned.

In a further aspect, the present disclosure provides a use for preventing or treating the mitochondrial dysfunction-related diseases of the pharmaceutical composition containing the mitochondria.

Then, the pharmaceutical composition, prevention, treatment, and mitochondrial dysfunction-related diseases are aforementioned.

Additionally, the present disclosure may provide a method for preventing or treating the mitochondrial dysfunction-related diseases, which includes administering the pharmaceutical composition containing the mitochondria as an active ingredient to a subject.

Then, the pharmaceutical composition, administration, administration dose, prevention, and treatment are aforementioned. The subject may be a patient suffering from a disease or an individual likely to have the disease. Then, the subject may be a mammal, and preferably a human.

The disease is a disease caused by a mitochondria dysfunction, and specific diseases are as described above.

In particular, the administration may be administered through an injection into the lesion, subcutaneous injection, or intravenous injection. Through this, the pharmaceutical composition according to the present disclosure may supply frozen platelet-derived mitochondria with normal activity directly to the lesion where the disease has occurred, thus being useful for increasing the activity of cells with deterioration in mitochondrial functions or for regenerating cells with mitochondrial dysfunction, and may be used for the prevention or treatment of the mitochondria-related diseases, specifically inflammatory diseases.

Hereinafter, the present disclosure will be described in more detail through the following Example. However, these Examples are for illustrative purposes of the present disclosure, and the scope of the present disclosure should not be limited to these Examples.

### Preparation Example 1. Preparation of platelets

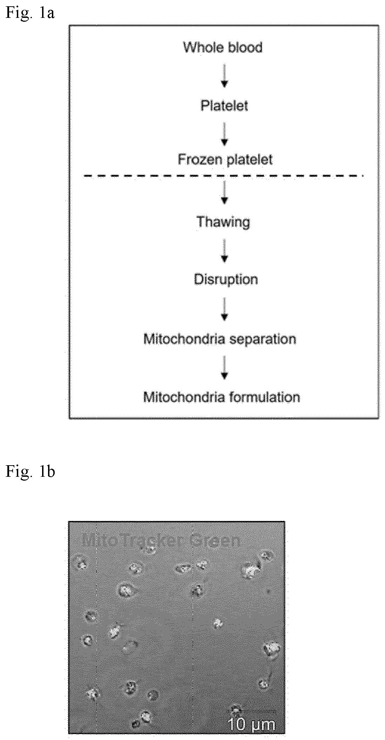

Whole blood containing K2-dipotassium ethylenediaminetetraacetic acid (EDTA) was centrifuged at 500 ×g for 3 minutes to obtain the supernatant containing platelet-rich plasma (PRP). The obtained supernatant was centrifuged at 2,000 ×g for 5 minutes to obtain a platelet precipitate (pellet) produced (FIG. 1a). The obtained platelet precipitate was resuspended in Dulbecco's phosphate-buffered saline (DPBS).

The obtained platelet precipitate resuspended in DPBS was fluorescently stained with Mitotracker Green, which is a mitochondria-specific green marker, and observed using a confocal microscope (FIG. 1b).

### Example 1. Obtaining mitochondria from platelets

### Example 1.1. Isolation of mitochondria of platelets without freezing and thawing

Thereafter, mitochondria were isolated from platelets (FIG. 1A). Specifically, the isolated platelet precipitate was physically disrupted using only a vortex and/or a 27G- or 29G syringe for 1 minute. After disruption of the platelets, the resultant was centrifuged at 2,000 ×g for 5 minutes. Then, the resulting supernatant was centrifuged at 12,000 ×g for 10 minutes to obtain precipitated mitochondria.

### Example 1.2. Isolation of mitochondria after performing freezing and thawing of platelets

Mitochondria were isolated from platelets in the same manner as in Example 1.1, but freezing and thawing were additionally performed before disruption of the platelets. Specifically, the platelet precipitate isolated by the method of Preparation Example 1.1 were each resuspended in a SHE mixed preservation solution (0.25 M sucrose, 20 mM HEPES buffer (pH 7.4), and 2 mM EGTA), a TTG mixed preservation solution (0.195 M trehalose, 20 mM Tris, and 65 mM glycine), and a TTGE mixed preservation solution (0.195 M trehalose, 20 mM Tris buffer, 65 mM glycine, and 2 mM EDTA). Thereafter, each resultant was quickly frozen using LN₂ or frozen at -80°C under conditions with or without using a freezing container (FC).

The frozen platelets were thawed at room temperature and the isolated platelet precipitate was physically disrupted using a vortex and/or a 27G or 29G syringe for 1 minute. After disruption of the platelets, the resultant was centrifuged at 2,000 ×g for 5 minutes. Then, the resulting supernatant was centrifuged at 12,000 ×g for 10 minutes to obtain precipitated mitochondria.

### Example 2. Confirmation of mitochondria functions according to whether platelets have been frozen and thawed

### Example 2.1. Confirmation of characteristics of mitochondria isolated without freezing and thawing of platelets

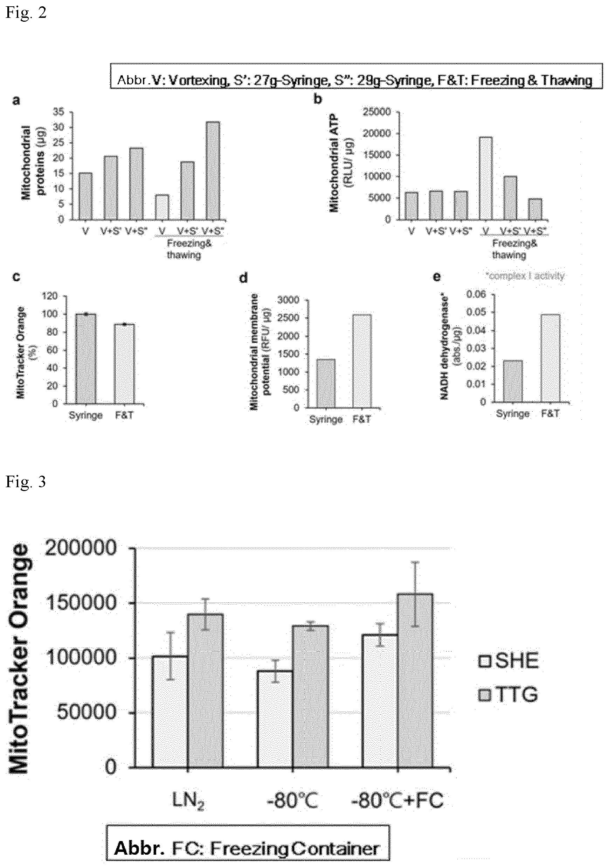

For the mitochondria obtained in Example 1.1, the concentration of mitochondrial proteins was measured using the bicinchoninic Acid (BCA) assay, and the amount of ATP present in mitochondria was confirmed using the CellTiter-Glo luminescence kit (Promega, Madison, WI, USA). In addition, the mitochondria obtained were fluorescently stained with Mitotracker Orange, which is a yellow marker specific to mitochondria, and observed with a confocal microscope, and the membrane potential of mitochondria was measured using TMRE (Invitrogen, cat no. T669) dye. Meanwhile, the activity of NADPH dehydrogenase (complex I) was confirmed using CCK-8 (Enzo Life science, Cat No. ALX-850-039) (FIG. 2).

### Example 2.2. Characterization of isolated mitochondria after performing freezing and thawing of platelets

The mitochondria isolated in the same manner as in Example 1.2 were confirmed by the method in Example 2.1. The results are shown in FIG. 2.

### Example 3. Confirmation of mitochondria functions according to isolation solution and freezing conditions

After resuspending the platelet precipitate obtained in Preparation Example 1 with a SHE mixed preservation solution and a TTG mixed preservation solution, respectively, the resuspended platelet precipitate was quickly frozen using LN₂ or the platelets frozen at 80°C under the condition of not using a freezing container (FC), and were fluorescently stained with Mitotracker Orange, which is a mitochondrial-specific yellow marker. The resultant was observed using a confocal microscope.

As a result, it was confirmed that the yield of mitochondria isolated from the platelets frozen in the trehalose-Tris-glycine (TTG) mixed preservation solution showed an increase by 44% compared to that of mitochondria isolated from the platelets frozen in the sucrose-HEPES-EGTA (SHE) mixed preservation solution. In addition, it was confirmed that the yield of mitochondria isolated from the platelets, which were slowly frozen at -80°C using a freezing container (FC), showed an increase by about 80% compared to that of mitochondria isolated from the platelets, which were quickly frozen using LN₂ (FIG. 3). Then, when the freezing container is used, the temperature is lowered to about -1°C per minute.

In addition, the platelet-derived mitochondria isolated after freezing the platelets in a mixed preservation solution (SHE or TTG) showed a higher amount of ATP retention compared to the amount of ATP retention by the syringe disruption method, and the enzyme activity of the membrane potential and oxidative oxidation complex 1 were also maintained at high levels.

### Example 4. Confirmation of mitochondria according to freezing storage period of platelets

The isolation yield and concentration of mitochondria according to the period of platelets were confirmed. In particular, the platelets frozen at -80°C using a freezing container in SHE mixed preservation solution were used.

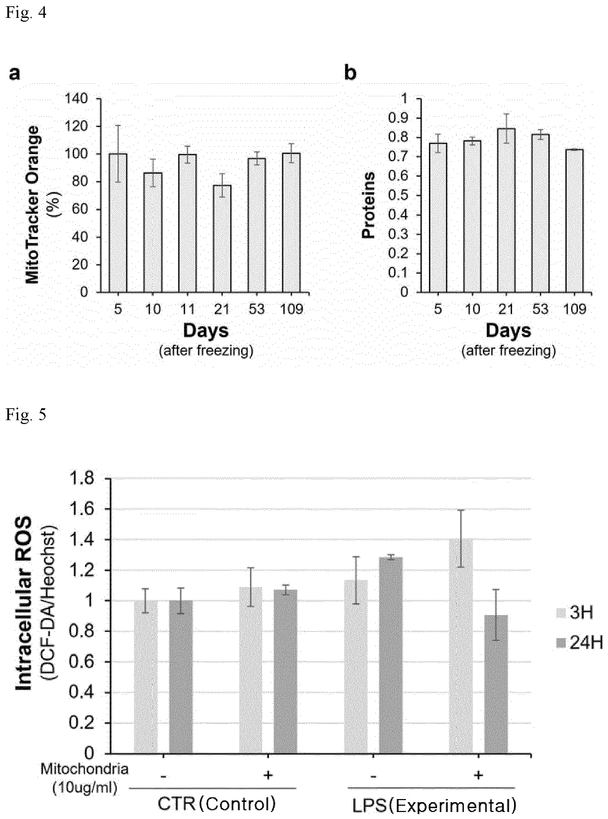

Specifically, platelets, which were stored frozen for 5 to 109 days, were fluorescently stained with Mitotracker Orange, a mitochondria-specific yellow marker, and measured with a fluorometer. The concentration of mitochondrial proteins was measured using the BCA assay. As a result, it was confirmed that the platelets which were stored frozen maintained the yield of isolated mitochondria even until day 109 (FIG. 4).

### Example 5. Confirmation of effect of platelet-derived mitochondria on inhibition of reactive oxygen species: In vitro

4 × 10⁵ THP-1 human mononuclear cells were cultured in RPMI 1640 medium containing 1% fetal bovine serum (FBS), and 16 to 24 hours thereafter, the platelet-derived mitochondria obtained in Preparation Example 2 were treated to the THP-1 cells. After 30 minutes of treatment, an inflammatory cell model was prepared by treatment with lipopolysaccharide (LPS) at a concentration of 2 µg/mL. 24 hours thereafter, the LPS-treated THP-1 cells were stained with dichlorofluorescin diacetate (DCF-DA), which is a cell membrane-permeable indicator of reactive oxygen species, and an amount of intracellular reactive oxygen species were measured. In addition, the number of cells was corrected by staining with Hoechst 33432, which is a nucleus-specific dye. The results are shown in FIG. 5.

### Example 6. Confirmation of anti-inflammatory effect of platelet-derived mitochondria: In vivo

After adapting 5 to 8-week-old male C57BL/6 mice for one week in the SPF zone, an experiment was performed to confirm the survival rate of an inflammatory animal model following the administration of platelet-derived mitochondria. During the acclimation period, a habitat for mice was prepared with a day and night cycle at intervals of 12 hours, and an indoor temperature of 23±2°C and a humidity of 40% to 60% were maintained.

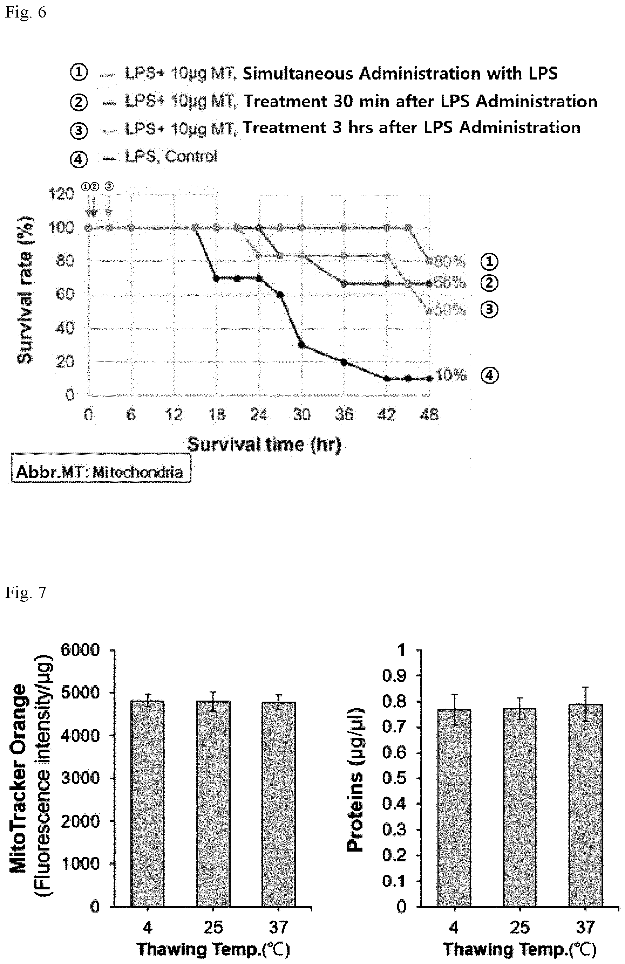

The mice that had passed the acclimation period were treated with LPS to prepare an inflammatory animal model. Then, 10 µg of platelet-derived mitochondria were administered to the experimental group by intravenous injection simultaneously, 30 minutes thereafter, or 3 hours thereafter. As a result of measuring the survival rate after administration, it was confirmed that the individual survival rate of the inflammation animal model was significantly improved by the administration of platelet-derived mitochondria. In particular, it was confirmed that when LPS and mitochondria were administered simultaneously, the survival rate was the highest to reach 80% (FIG. 6). In particular, for the mitochondria administered to the experimental group, mitochondria isolated from platelets frozen at -80°C using a freezing container in the SHE mixed preservation solution were used.

### Example 7. Comparison of mitochondrial isolation yield according to thawing conditions of frozen platelets

The separation yield and concentration of mitochondria according to the period of thawing and storage of frozen platelets were confirmed. Then, platelets were used as platelets frozen at -80°C using a freezing container (FC) in SHE mixed preservation solution.

Specifically, platelets stored frozen for two months were thawed at 4°C, 25°C, or 37°C, mitochondria were isolated, and fluorescently stained with Mitotracker Orange, a specific yellow marker, and measured with a fluorometer (Fig. 7 left). The concentration of the mitochondrial protein was measured using the BCA method (Fig. 7 right). As a result, it was confirmed to have a similar separation yield without affecting the temperature conditions during thawing.

Although a method for obtaining platelet-derived mitochondria and use thereof have been described with reference to the specific embodiments, they are not limited thereto. Therefore, it will be readily understood by those skilled in the art that various modifications and changes may be made thereto without departing from the spirit and scope of the present disclosure defined by the appended claims.

## Claims

1. A method for stably storing mitochondria by freezing platelets.

2. A method for obtaining platelet-derived mitochondria, comprising:
thawing frozen platelets; and
disrupting the thawed platelets.

3. The method of claim 2, further comprising removing the lysate excluding mitochondria.

4. The method of claim 2, wherein the thawing is performed at 0°C to 37°C.

5. The method of claim 2, wherein the thawing is performed for 1 minute to 1 hour.

6. The method of claim 3, wherein the removing the lysate is performed by centrifugation, or using a filter, a mesh, or beads.

7. The method of claim 2, wherein the frozen platelets are those obtained by freezing the platelets obtained from a subject.

8. The method of claim 7, wherein the freezing is performed at a temperature in the range of -1°C to -200°C.

9. The method of claim 2, wherein before disrupting the platelets, freezing and thawing of platelets are further performed at least once.

10. A pharmaceutical composition for preventing or treating diseases, comprising the platelet-derived mitochondria obtained according to any one obtaining method of claims 2 to 9 as an active ingredient.

11. The pharmaceutical composition of claim 10, wherein the disease is a mitochondrial dysfunction disease selected from a group consisting of chronic inflammatory disease, acute inflammatory disease, ischemic disease, neurological disease, heart disease, muscular disease, degenerative disease, metabolic disease, fibrotic disease, joint disease, eye disease, hair loss, and immune-related disease.

12. A use of a platelet-derived mitochondria obtained according to any one obtaining method of claims 2 to 9 for the manufacture of a medicament for preventing or treating a mitochondria dysfunction-related disease.

13. A use of a pharmaceutical composition of claim 10 for preventing or treating a mitochondria dysfunction-related disease.

14. A method of preventing or treating a mitochondria dysfunction-related disease comprising:
administering a pharmaceutical composition of claim 10 to a subject.
